# EUROPEAN PATENT APPLICATION

(11) **EP 2 474 239 A1**
(43) Date of publication of application: **11.07.2012**
(21) Application number: 12002482.3
(22) Date of filing: 30.05.2008
(51) Int. Cl.: A23L 1/29, A61P 3/00, A61P 3/04, A61P 3/10, A61P 9/00, A23L 1/30

(54) **Kit of nutritional compositions for infants with fat and carbohydrate content adapted to the particular stage of development**

(30) Priority: 30.05.2007 US 755589; 07.09.2007 EP 07017568
(62) Divisional of application: 08758882.8
(71) Applicant: Nestec S.A., 1800 Vevey (CH)
(72) Inventor: Shahkhalili, Yasaman, 1814 La-Tour-de Peliz (CH); Acheson, Kevin, 1037 Etagnières (CH); Mace, Katherine, 1005 Lausanne (CH); Moulin, Julie, 1616 Attalens (CH); Zbinden, Irene, 1052 Le Mont sur Lausanne (CH); Aprikian, Olivier, 1800 Vevey (CH); Voss, Theresa, 1818 La-Tour-de Peilz (CH); Ursel, Silja, 78467 Konstanz (DE); Kuslys, Martinas, 3506 Grosshochstetten (CH)
(74) Representative: Lock, Graham James

(57) **Abstract**

The present invention generally relates to the field of nutrition. In particular the present invention relates to infant nutrition in the post natal period and in early life, more particular during the age period of 6-36 months or during a part thereof. One embodiment of the present invention is a kit of diet compositions for children during the age period of 6-36 months or during a part thereof, wherein the macronutrient content of the compositions is gradually changing in the form of a straight line from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months, and its use to prevent obesity later in life. Another embodiment relates to a range of shelf-stable baby food products having an optimal energy profile for meeting the nutritional needs of an infant at a specific stage of infant development.

## Description

### BACKGROUND

The present invention generally relates to the field of nutrition. In particular the present invention relates to infant nutrition in the post natal period and in early life, more particular during the age period of 6-36 months or during a part thereof. The present invention also relates to shelf stable baby foods and particularly to shelf stable baby foods which have an optimum energy profile for meeting the nutritional needs of an infant.

### SUMMARY

Evidence is accumulating that nutrition during early life can program the development of diseases later in life¹, a discovery that was named metabolic programming or imprinting". This evidence, mainly driven from foetal development in-utero²⁻⁷, reveals the importance of optimal nutrition during early life for the health of the individual later in the life. Considering that many developmental processes still continue during early post natal life, it is evident that postnatal nutrition - especially during suckling and during the complementary feeding period - plays an important role for the health status and for the prevention of diseases later in life.

Prior evidence in rats demonstrates that a change in the fat and carbohydrate (CHO) content of milk during the suckling period may have an impact on the development of obesity and diabetes later in life. In these studies⁸⁻⁹ that relate to breast feeding without additives rats were artificially reared by using either a milk substitute formula low in fat content (LF) and rich in CHO (20% total energy from fat (fat E) and 56% total energy from carbohydrates (CHO E), respectively) or by using a milk composition similar to rat milk, namely high in fat (HF) and low in CHO (8% CHO E and 68% fat E) or were mother-fed from the age of 4 to 24 days postnatal. All groups were weaned onto a low fat laboratory standard chow diet. The LF feeding during the suckling period resulted in hyperinsulinemia which persisted into adulthood and lead to an increase in body weight and onset of adult obesity, an effect termed "metabolic programming"¹⁰⁻¹¹Beyond total milk feeding (suckling period) this effect was previously not investigated to the inventor's best knowledge.

Although during the suckling period "milk" as the first diet of infants and other mammals is very rich in fat (50% of energy from fat), the dietary fat intake is reduced considerably during the complementary feeding period as an infant is gradually weaned off milk onto semi solid foods.

This is due to the replacement of high-fat milk with weaning foods low in fat content, such as fruits, vegetables, weaning cereals, fruit juices etc.

It has been reported that the fat intake of infants, even in developed countries, is low (30% of energy from fat) during the complementary feeding period (6-12 months)¹²⁻¹³. Indeed the complementary feeding period has been referred to as "the period of life with the lowest fat intake during the life cycle of man".

Data concerning nutritional recommendations for humans during the weaning period are scarce and recommendations are mainly based on estimates of the nutritional requirements of those of suckling infants adjusted for weight and energy intake. Infant nutrition during this period of rapid growth is surrounded by uncertainties and there is little agreement about what should constitute an optimal composition of the complementary diet and in particular an optimal fat and carbohydrate content of complementary diets.

Selecting appropriate daily requirements for infants and young children can be very challenging, because different information, based on different methodologies and age groups exists worldwide. There is, therefore, a need for novel products and methods for addressing the daily nutritional needs of developing infants.

The present uncertainty about the long-term consequences of the fat and CHO content of the weaning diet on health in general and - in particular - on development of obesity later in life led the present inventors to investigate these consequences in rats as model system for humans.

It is an advantage of the present invention to provide a nutritional concept for the transition period from nutrition with breast milk or breast milk-like products in terms of fat and CHO content during the sucking period to the subsequent nutrition with baby food that allows it to reduce the risk that the child develops a bad health status, in particular obesity and diabetes later in life.

The present invention provides kits, methods of reducing the risk of obesity and mealplans.

In one embodiment the present invention relates to a kit of parts comprising diet compositions for children during the age period of 6-36 months or during a part thereof. In an embodiment, the macronutrient content of the compositions is gradually changing in the form of a straight line from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months.

A wide variety of kits and methods of using the kit are possible and envisioned by the present invention.

It is also an object of the invention to provide a range of shelf stable baby food products which meet the nutritional needs of developing infants.

It is another object of the present invention to provide a feeding regime for meeting the daily nutritional needs of developing infants.

It is a further object of the invention to provide methods and uses for the prevention or treatment of nutritionally related disorders in infants and toddlers.

These and other objects are achieved by providing a range of shelf stable baby load products having an optimal energy profile for meeting the nutritional needs of an infant through a number of stages of infant development, wherein two or more baby food products from the range may be combined to provide an optimal energy profile for meeting the nutritional needs of an infant at a particular stage of infant development. In one embodiment, the stages of infant development are selected from (i) from about 4 to about 6 months of age (Stage 1); (ii) from about 6 to about 8 months of age (Stage 2); (iii) from about 8 to about 12 months of age (Stage 3); and (iv) from about 12 to about 36 months of age (Stage 4 or Stage Junior).

Additional features and advantages are described herein, and will be apparent from, the following Detailed Description and the figures.

### BRIEF DESCRIPTION OF THE FIGURES

Figure t shows the % energy content of fat (grey lines) and carbohydrates (black lines) of the total energy content of the diet composition depending on the age of the child in month. The x-axis shows the age of the child in month and the y-axis shows the %-energy content of the diet composition.

Figure 2 shows the energy intake during all study phases, phase I, phase II, phase III and the overall energy intake of Experiment I.

Figure 3 shows the development of the body weights of thc test animals during each study phase, phase I, phase II and phase III during Experiment I.

Figure 4 shows the weight gain of the different groups of test animals during high fat period (Phase III) in Experiment I.

Figure 5 shows the fat gain of the different groups of test animals during high fat period (Phase III) in Experiment I.

Figure 6 illustrates an example of a feeding plan which provides a suggested daily feeding schedule for each stage of infant development.

### DETAILED DESCRIPTION

% energy refers for the purpose of the present invention always to the total amount of energy that is present in a diet composition. A diet composition that has 33% energy from fat will therefore have 67% energy from carbohydrates and/or proteins.

A diet composition means at least one meal or a part thereof. For example, a diet composition can be a complete meal such as breakfast, lunch or dinner. It can also be one or more, e.g. five, individual meals that are consumed during the day. It can also be more than one individual meal. It can also represent only a part of an individual meal or a part of more individual meals. It is preferred that the diet compositions of the present invention represent individual meals: Oftentimes dinner represents a substantial part of the diet on a caloric basis. In infants, the caloric intake during dinner can range from .15 to 35% of the daily caloric intake. Hence, in a preferred embodiment the meals are dinners. Dinners are the main meat of the day and can be served in the evening or at midday. Nutritionally well-balanced dinners can, hence, significantly contribute to the health of infants.

Macronutrients arc those nutrients that together provide most metabolic energy to an organism. For the purpose of the present invention the three macronutrients are carbohydrates, proteins, and fats.

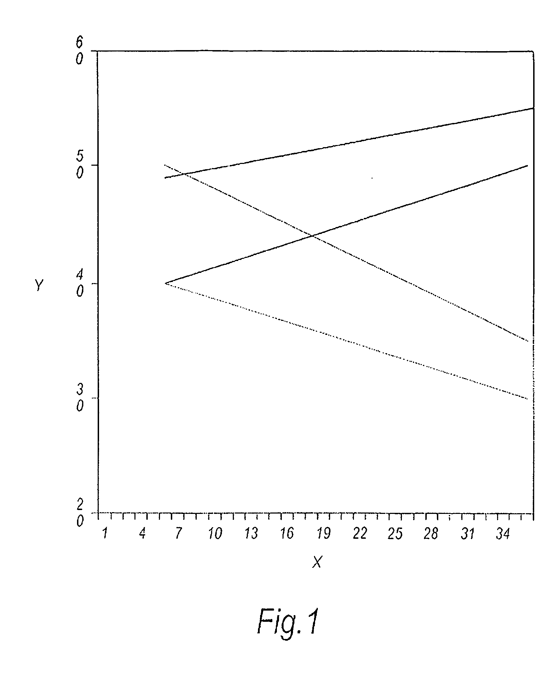

Gradually changing in the form of a straight line from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months means any straight line or group of straight lines that is located on and/or between the two black lines in Figure 1 for carbohydrates and any straight line that is located on and/or between the two grey lines in Figure 1 for fats. The present invention comprises all possible kits of diet compositions that are represented by Figure 1 and all such kits of diet compositions are disclosed by Figure 1.

Expressed in mathematical terms this means that the present invention discloses and comprises every kit of diet compositions for children during the age period of 6-36 months or during a part thereof, wherein the fat content of the diet composition is adjusted to any straight line, which is located on or between the straight lines

y= -1/2 x + 53 and y=-1/3x + 42 (y is % energy from fat), and

wherein the carbohydrate content of the diet composition is adjusted to any straight line, which is located on or between the straight lines

y= 1/5 x + 47.8 and y=1/3x + 38 (y is % energy from carbohydrates)

between x=6 and x=36, if x is the age of the child in months.

A kit of parts in accordance with the present invention provides a tool for the to improve infant nutrition in an optimal way without having to keep detailed records of the nutritional components that the infant has already ingested.

The present method and kit thus increase convenience and reduce the occurrence of consequences of non-optimal nutrition.

"Infants" as used in the present invention are in particular human children aged from 4 months to 3 years. The present method is particularly directed at infants aged from 6 to 36 months.

The term "about" means plus or minus 20%, more preferably plus or minus 10%, even more preferably plus or minus 5%, most preferably plus or minus 2%.

The term "food product" means any food, feed, snack, food supplement, treat, meal substitute, or meal replacement, whether intended for a human or an animal.

The term "%E" means the percentage of total daily energy intake.

The term "complete meal" means a meal that is designed to provide one nutritionally balanced serving, i.e. it is not necessary to combine the complete meal with another food product to provide a meal.

Complementary foods are used for weaning and they can be defined as "any food, whether manufactured or locally prepared, suitable as a complement to breast milk or to infant formula, when either becomes insufficient to satisfy the nutritional requirements of the infant." They include, for example, milk products, home made foods and processed foods (cereal-based or other baby foods, including ready-to-eat preparations).

Complementary foods should be introduced into an infant's diet when breast-milk or a breast-milk substitute no longer satisfies the infant's nutritional requirements. During this transition period, as the infant's digestive system develops, the infant's diet can gradually evolve from an exclusive milk diet to a fully diversified diet similar to that of adults.

The term "CHO" means carbohydrate.

The term "LA" means linoleic acid.

The term "ALA" means alpha linolenic acid.

The term "shelf stable baby food product" means a baby food product that can be safely stored and sold in a sealed container at room temperature while still having a useful shelf life, for example at least about 2 months, preferably longer.

In one embodiment of the present invention the diet compositions are daily diet compositions. Daily diet compositions have the advantage that they can be used very effectively to tightly regulate the gradual change of the fat and carbohydrate content of the food of a child when it is gaining age. It is important to note that if the diet compositions are daily diet compositions the individual meals may well deviate somewhat from a macronutrient content that is gradually changing in the form of a straight line from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months, as long as the individual meals to be consumed during a day together - that together are considered a daily diet composition - fulfil this requirement.

This way it is possible to adapt a mealplan of a child, e.g., in a way that it can consume food that is easy to digest in the evening to allow an easy sleep, while it consumes food compositions that are more difficult to digest during the day.

In one embodiment of the present invention the total energy content of the composition is gradually changing in the form of a straight line from a composition that comprises about 670-715 kcal/day for children at the age of 6 months to a composition that comprises about 1000-1200 kcal/day for children at the age of 36 months. This allows adapting the energy content of the diet compositions to the particular age of the child, so that it can be assured that a sufficient amount of energy is always present during this decisive period of development of the child, while an overfeeding is avoided.

Changing gradually means in one embodiment of the present invention that the fat and carbohydrate content of the diet composition is adjusted daily, based on the age of the child and the corresponding optimal fat content and carbohydrate content of the composition. Optionally, the total energy content is also adjusted daily along with the fat and carbohydrate content. This results in a very gradual change without any noticeable "steps" in diet composition. Consequently, the infant's organism will not have to adapt to any abrupt changes in food content.

In another embodiment of the present invention changing gradually means that the content of the diet composition is adjusted weekly, based on the age of the child and the corresponding optimal fat content and carbohydrate content of the composition, Also in this case the changes that are made to the diet composition are so marginal that the metabolism of the infant will not he faced with any abrupt changes.

In another embodiment of the present invention changing gradually means that the content of the diet composition is adjusted monthly, based on the age of the child and the corresponding optimal fat content and carbohydrate content of the composition. Even if the adjustment of the diet composition is made on a monthly level the resulting mealplan of a child will exhibit a smooth transition from a high fat-low carbohydrate composition at the age of 6 months to a low fat - high carbohydrate composition at the age of 36 months without any abrupt changes.

The present inventors have found that the object of the present invention can still well be achieved by adjusting the macronutrient content of the diet composition stepwise, based on the age of the child and the corresponding optimal fat content and carbohydrate content of the composition as detailed above, preferably in 3-10 steps, most preferred in 3-4 steps during the age of 6-36 months.

This stepwise adjustment is preferable made at a child age of 6, 8, 12, 18, 24 and/or 36 months.

In one preferred embodiment of the present invention changing gradually includes that the content of the diet composition is adjusted also with respect to the total calorie content based on the age of the child and the corresponding optimal calorie content of the composition.

For example, a kit according to the present invention can comprise at least one diet composition that comprises about 44-46% energy from fat and about 47-49% energy from carbohydrates during the age of 6-8 months, at least one diet composition that comprises about 39-41% energy from fat and about 49-52% energy from carbohydrate during the age of 8-12 months, and/or at least one diet composition that comprises about 34-35% energy from fat and about 51-53% energy from carbohydrate during the age of 12-36 months. Preferably, the at least one diet composition for consumption during the age of 6-8 months comprises about 670-715 kcal/day, the at least one diet composition for consumption during the age of 8-12 months comprises about 715-850 kcal/day, and/or the at least one diet composition for consumption during the age of 12-36 months comprises about 750-1200 kcal/day.

The diet compositions of the present invention further comprise a protein source. The amount of protein source present is preferably adjusted to the need of the child of the particular age in question and can generally be calculated as follows:

% energy from protein = 100 - (% energy from fat + % energy from carbohydrates)

In a particular prefered embodiment of the present invention the content of carbohydrates in the compositions is gradually changing from a composition that comprises about 48% energy from carbohydrates for children at the age of 6 months to a composition that comprises about 53% energy from carbohydrates for children at the age of 36 months.

Generally the kit of the present invention comprises at least one, preferably at least two diet compositions. A diet composition preferably constitutes one or more complete meals, one or more parts of a complete meal or one or more snacks or a part thereof.

The number of diet compositions the kit of the present invention can contain is not particularly limited and is only regulated by the storage stability of the food product. Hence, a kit intended for use in a nursery or in a hospital can be significantly larger than a kit for private households.

In a preferred embodiment the kit of the present invention comprises diet compositions for at least one day. In this respect the kit might comprise 2 or more, preferably 3 - 10, even more preferred 5 individual meals. The daily food intake can, e.g., be divided into three meals and 2 up to 3 snacks. More meals with corresponding smaller portions have the advantage that the child's metabolism will not be faced with too large amount of food and at the same time periods with an "empty stomach" are avoided.

In further embodiments of the present invention the kit comprises diet compositions for three days, for a week or for a month. It is preferred that the diet compositions represent one or more individual meals. In this case the kit can for example comprise at least 3, preferably 3-21, most preferred 9 individual meals.

It is further preferred that the diet compositions represent a total diet, which is to be understood as sum of food to be consumed by a person over a given period of time.

Preferably, the diet composition of the present invention also comprises micronutrients and/or minerals to arrive at an ideally balanced food product for the child at the particular age. Also these components can preferably be varied based on the specific needs of the child at a particular age.

In a preferred embodiment of the present invention the fat component comprises essential fatty acids. Essential fatty acids are fatty acids that cannot be produced by the body. They are capable of fulfilling important functions in the human body. Two families of essential fatty acids are in particular important, the omega-3 and the omega-6 family. Alpha-linolenic acid (ALA, a fatty acid with a chain length of 18 carbon atoms and containing three double bonds) is an example of a member of the omega-3 family. ALA is, e.g., found in flaxseed and various vegetable oils and nuts. An example of a member of the omega-6 family of essential fatty acids is linoleic acid (LA, a fatty acid with a chain length of 18 carbon atoms and containing two double bonds). The weight ratio of omega-6/omega-3 fatty acids in the diet compositions of the present invention is preferably between 5 and 15.

Preferably, the diet compositions of the present invention are of a liquid nature. Preferred embodiments have a consistency of a liquid or of a mash. This can be achieved by the presence of water in the diet compositions of the present invention. Preferably, they contain between 75 and 90 wt-% water based on the total weight of the diet composition, more preferably between 78 and 85 wt-% water.

In a preferred embodiment, the diet compositions to be administered to the infants each have a volume between 90 and 500 ml, more preferably between 125 and 300 ml. The diet compositions of the present invention may all have about the same volume (i.e. difference between greatest and smallest volume is less than 50 ml) or they may have increasing volumes with the increasing age of the child to reflect an increased caloric content.

Preferably, the diet compositions of the present invention are to be consumed at a temperature of between 15 and 55°C, more preferred between 30 and 50°C, and even more preferably between 35 and 45°C.

The diet compositions according to the present invention are preferably individually packaged and provided as a kit of parts. The kit of parts contains in one embodiment several different meals. The diet compositions in the kit arc preferably in ready-to-eat and/or dried form. From the dried form, a ready-to-eat form can be easily produced by reconstitution in a suitable liquid, e.g. water. The ready-to-eat form can normally be administered directly to the infant, optionally after heating and/or mixing.

The present invention relates also to the use of a series of diet compositions, wherein the macronutrient content of the compositions is gradually changing from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months for the preparation of a kit to prevent the development of obesity.

The kit that is to be prepared by the use of the present invention can be any kit of the present invention and can have any feature or any combination of features as described herein.

The series of diet compositions comprises 2 or more individual diet compositions, preferably 3-35 diet compositions.

The present invention also relates to a mealplan for children that comprises a kit of the present invention. The mealplan is intended for the prevention of obesity later in life.

In another aspect, the invention provides a range of shelf stable baby food products having an optimal energy profile for meeting the nutritional needs of an infant at a specific stage of infant development, wherein two or more baby food products from the range may be combined to provide an optimal energy profile for meeting the daily nutritional needs of an infant at a particular stage of infant development.

In one embodiment, the stages of infant development are selected from: (i) from about 4 to about 6 months of age (Stage 1); (ii) from about 6 to about 8 months of age (Stage 2); (iii) from about 8 to about 12 months of age (Stage 3); and (iv) from about 12 to about 36 months of age (Stage 4 or Stage Junior).

Preferably, the range of products comprises at least one food product suitable for that stage of infant development.

Preferably, the range of products comprises two or more food products suitable for each stage of infant development. More preferably, the range of products comprises at least a vegetable based product and a fruit based product for each stage of infant development. More preferably, the range of products comprises a plurality of vegetable based products and fruit based products for each stage of infant development.

Preferably, the range of products comprises art least a vegetable based product and a fruit based product for stage 1 of infant development.

Preferably, the range of products comprises at least a vegetable based product, a complete meal and a fruit based product for stage 2 of infant development.

Preferably, the range of products comprises at least a vegetable based product, a complete meal and a fruit based product for stage 3 of infant development.

Preferably, the range of products comprises at least a vegetable based product, a complete meal and a fruit based product for stage 4 of infant development.

In one embodiment, the total optimum daily energy intake for stage 1 is between about 530 and about 700 kcal/day, preferably about 625 kcal/day, for stage 2 is between about 620 and about 720 kcal/day, preferably about 670 kcal/day, for stage 3 is between about 680 and about 930 kcal/day, preferably about 770 kcal/day, and for stage junior is between about 810 and about 1180 kcal/day, preferably about 1040 kcal/day.

Conveniently, two or more baby food products from the range may be combined with the daily milk intake of an infant to provide an optimal daily energy intake of an infant at a particular stage of infant development.

In preferred embodiments, two or more baby food products from the range provide a daily energy intake for stage I of between about 68 and about 238 kcal/day, preferably about 167 kcal/day, for stage 2 of between about 158 and about 258 kcal/day, preferably about 243 kcal/day, for stage 3 of between about 345 and about 595 kcal/day, preferably about 448 kcal/day, and for stage junior of between about 475 and about 845 kcal/day, preferably about 693 kcal/day.

Advantageously, the difference between the daily energy intake provided by the two or more baby food products and the optimum daily energy intake for each stage of infant development is provided by the daily milk intake of an infant.

In another example, the range of shelf stable baby food products has an optimal nutrient profile for meeting the nutritional needs of an infant through a number of stages of infant development.

Infants and young children's energy requirements per kg of body weight are 2 to 3 times that of adults. Since the food volume that an infant is able to ingest is limited in a context of large energy needs, an appropriate energy density of complementary foods plays an important role in determining the total energy intake. Energy density is defined as the amount of calories in a quantity of food or meal and is a good index of the true energy value of the different foods or meals. An optimal energy density is desired, since low levels may result in an energy deficit and poor growth and higher levels may be responsible for excessive energy intakes and subsequent weight gain, potentially leading to overweight later in life.

Energy intake in infants may be influenced by several factors. First, energy intake can increase through consumption of high energy density complementary foods, high milk intake and frequent meals and conversely, may decrease if viscous/bulky foods are consumed. Foods with a high energy density include meat and fatty fish, while viscous and bulky carbohydrate-based staples or porridges have a low energy-density. More specifically, energy density increases with the addition of fat and sugar and decreases with excessive water content. Finally, total energy intake may he influenced by the functional gastric capacity closely associated to the volume of food an infant can ingest during one meal, estimated at about 30 g/kg body weight. For infants and young children at 6 to 8, 9 to 11 and 12 to 23 months of age, this value corresponds to 243, 276 and 369 g/meal, respectively.

If meals are provided many times during the day, energy requirements can be satisfied with diets having lower energy density. Inversely, if the energy density of the complementary diet is high, daily energy needs can be covered by fewer meals.

In one embodiment, a daily intake of baby food products from the range has an average energy density of at least about 0.6 kcal/g, preferably at least about 0.67 kcal/g, most preferably about kcal/g.

The main advantage of providing fewer meals with a higher energy density lies in the fact that the complementary foods will be less likely to interfere with the milk intake.

With an average energy density of 1 kcal/g, infants and young children in all age groups should be able to cover their daily energy needs if they consume at least three meals per day. Taken separately, infants in stages 1 and 2 would have to consume 1 to 2 meals and infants and toddlers in stages 3 and 4 would have to take at least 2 to 3 meals per day. With a lower energy density of 0.7 kcal/g, the minimum number of daily meals would increase to 2 to 3 for Stage 2, 3 to 4 for Stage 3 and 3 to 5 for Stage 4. With lower energy densities, such as 0.5 kcal/g, a more frequent and difficult to achieve feeding frequency is necessary to attain the daily energy requirements from complementary foods. For example Stage 1 would require 2 to 3 meals per day, Stage 2 would require: 3 to 5 meals per day, Stage 3 would require 4 to 5 meals per day, and Stage 4 would require 5-6 meals per day.

In a preferred embodiment, one or more daily feeding schedules are provided which comprise suggested combinations of baby food products from the range of baby food products, said combinations providing an optimal energy profile for meeting the nutritional needs of an infant at a particular stage of infant development. Preferably, one or more daily feeding schedules are provided for each stage of infant development.

In one embodiment, the total optimum daily fat intake for stage 1 is about 45%E, for stage 2 is between about 43%E and about 45%E, for stage 3 is between about 40%E and about 43%E, and for stage junior is between about 30%E and about 40%E.

In one embodiment, two or more baby food products from the range provide a daily fat intake for stage 1 of between about 18%E and about 27%E, for stage 2 of between about 22%E and about 29%E, for stage 3 of between about 21%E and about 36%E, and for stage junior of between about 22%E and about 40%E.

In another embodiment, the difference between the daily fat intake provided by the two or more baby food products and the optimum daily fat intake for each stage of infant development is provided by the daily milk intake of an infant.

In preferred embodiments, the baby food products have a protein density of between about 1.5g/100kcal and about 3.2g/100kcal, preferably about 1.5g/100kcal.

In another embodiment, the baby food products comprise complete meals or incomplete meals. Preferably, a complete meal comprises two or more incomplete meals.

In one embodiment, each baby food product comprises a serving size of between about 65g and about 350g. Preferably, each baby food product comprises a serving size of between about 65g and about 100g for stage 1, of between about 65g and about 100g for stage 2, of between about 130g and about 200g for stage 3, and between about 130g and about 350g for stage junior.

In another aspect, the invention provides a feeding regime for meeting the nutritional needs of an infant during different stages of infant development, the regime comprising administering to an infant a range of baby food products as hereinbefore described.

In a further aspect, the invention provides use of a range of baby food products as hereinbefore described in the manufacture of a medicament for the prevention or treatment of a nutritionally related disorder in an infant.

In another aspect, the invention provides a method of preventing or treating a nutritionally related disorder in an infant, comprising administering to an infant a range of baby food products as hereinbefore described.

Preferably, the nutritionally related disease is selected from obesity, malnutrition, diabetes and heart disease.

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, devices, and materials are described herein.

All patents, patent applications, and publications mentioned herein are incorporated herein by reference to the extent allowed by law for the purpose of describing and disclosing the compounds and methodologies reported therein that might be used with the present invention. However, nothing herein is to be construed as an admission that the invention is not entitled to antedate such disclosure by virtue of prior invention.

All percentages for weights expressed herein are by weight of the total food product unless specifically stated otherwise.

Those skilled in the art will understand that it is possible to freely combine features and embodiments of the present invention as described herein without departing from the scope of the present invention as disclosed.

By way of example and not limitation, examples of the present invention will now be given.

Example 1:

Experiment 1

The consequence of fat and CHO content of weaning diet on the later development of obesity was investigated using rats.

Seventy two male Sprague-Dawley rats were separated from their dam at the age of 16 days. The animals were divided into three study groups (24 animals/group) and were pair-fed, on an iso-energetic and iso-protein basis, with one of the following weaning diets (Table 1) differing only in energy distribution from Fat and CHO as: (% energy) 10/70 (group A); 30/50 (group B) and 60/20 (group C). for 20 days (Phase 1: age 16 to 36 days). All groups were then fed *ad libitum* with a standard low-fat commercial chow diet (Kliba 3434, 13% fat E:) for 20 weeks (phase II: age 5 to 25 weeks), after which all groups were challenged with a high-fat diet (45% fat E: Kliba 2126) that was fed *ad-libitum* for a period of 18 weeks (phase III: age 35 to 53 weeks).

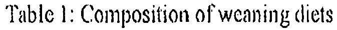

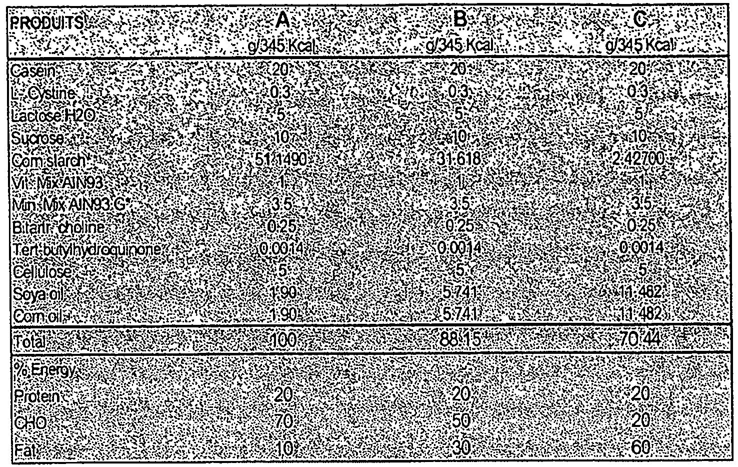

Food intake was measured daily during the weaning period (period 1) and twice per week during the post-weaning periods (period II &III). Body weight was measured 2-3 times per week throughout the study.

The body composition, which is body fat and fat free mass, was measured during post weaning phases II and III using NMR imaging (EchoMRI™ 2004), at 27, 35, 47 and 52 weeks of age.

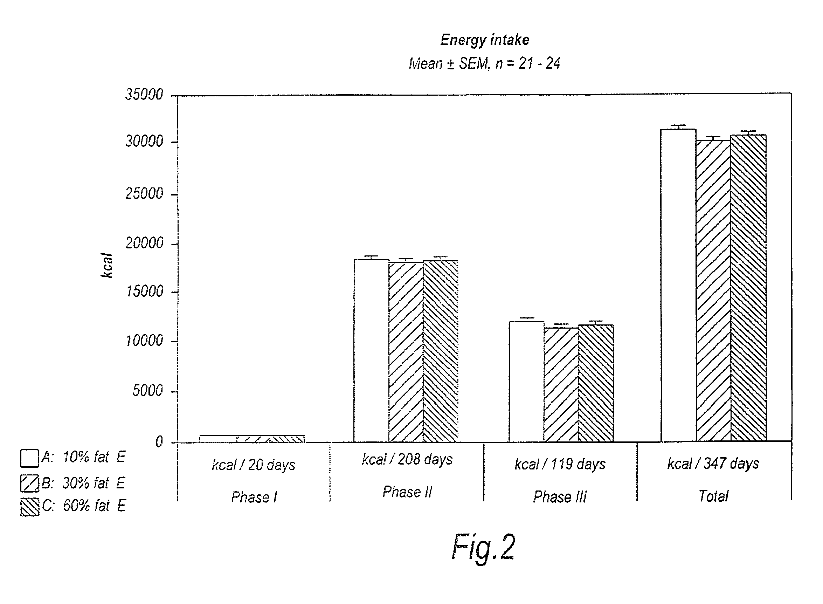

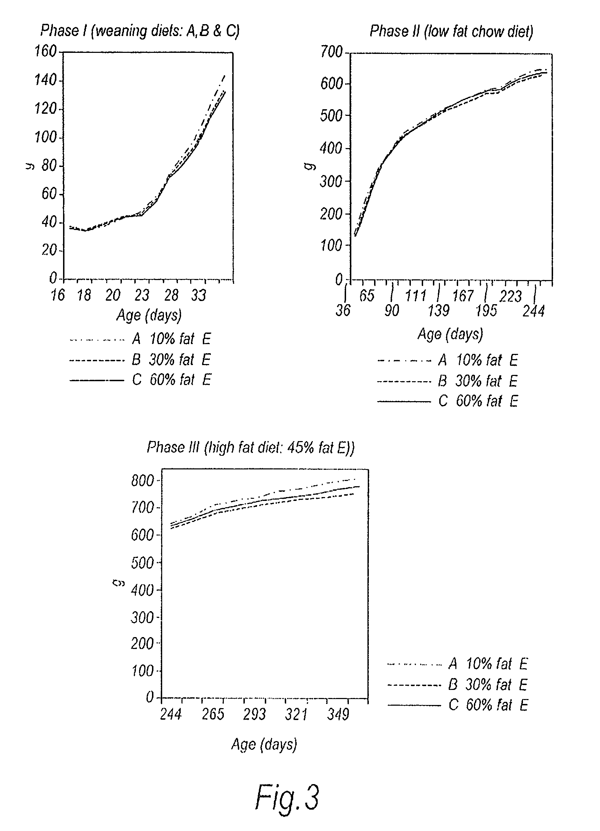

Figures 2-3 demonstrate that the energy intakes and body weights of all groups fed the weaning diets with different Fat/CHO ratios were similar at the end of the weaning period (phase 1), during 9 months of low-fat diet (phase II) as well as during the 4 months of the obesigenic, high-fat challenge (45% fat E) feeding period, (Phase III).

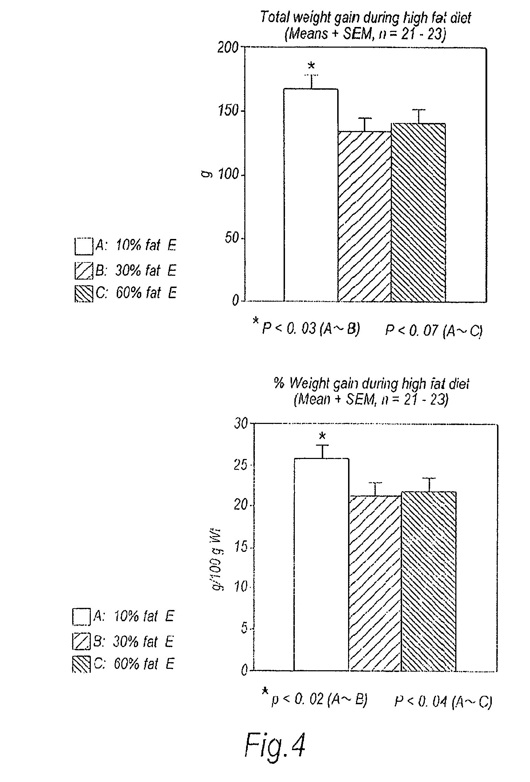

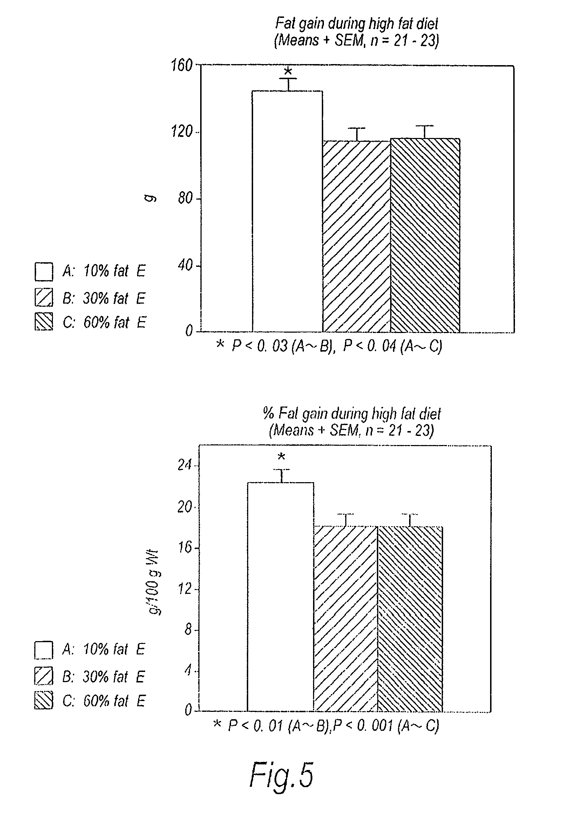

However, the rats fed with a low-fat (10% E), high-carbohydrate (70% E), diet only during 19 days weaning period gained significantly more body weight (Figure 4) and body fat (Figure 5) during the high-fat challenge diet at adult age (age of 3.5 to 53 weeks), relative to the other 2 groups fed with higher fat (30% & 60% E) and lower CHO (50% or 20% E) diets only during weaning period (p<0.05).

These results show that a weaning diet with a macronutrient composition (fat and CHO) close to that consumed during the suckling period (high fat diet) has a beneficial effect toward reducing the risk of development of obesity later in life, while a high-CHO, low-fat diet during the weaning period increases the susceptibility to excess body weight and fat mass gain later in life.

The inventors believe that this is the first report on "metabolic programming" during the complementary feeding period and reveals the importance of the Fat/CHO content of complementary diet for obesity prevention later in life.

In the following sample diet compositions are provided for the age period of 6-8 months (* = carbohydrates as monosaccharides):

Menu 1:

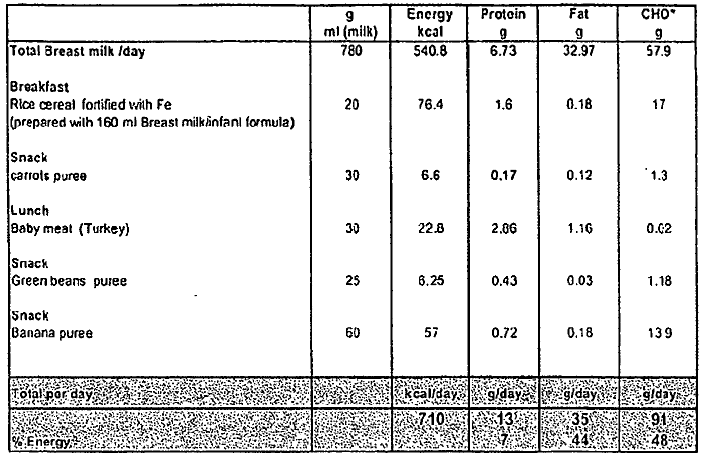

Menu 2:

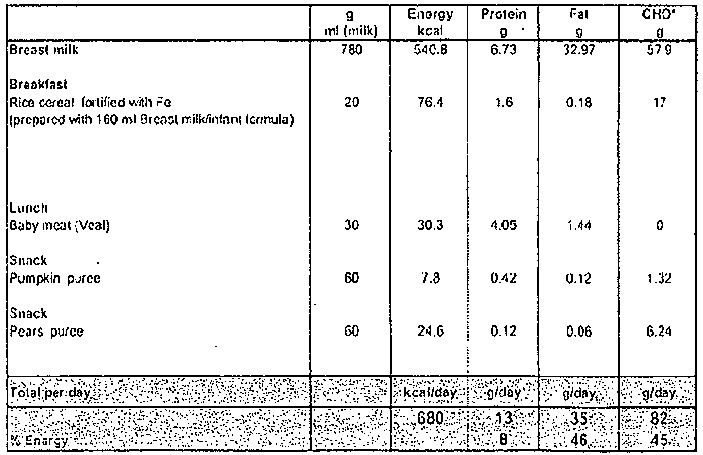

Menu 3:

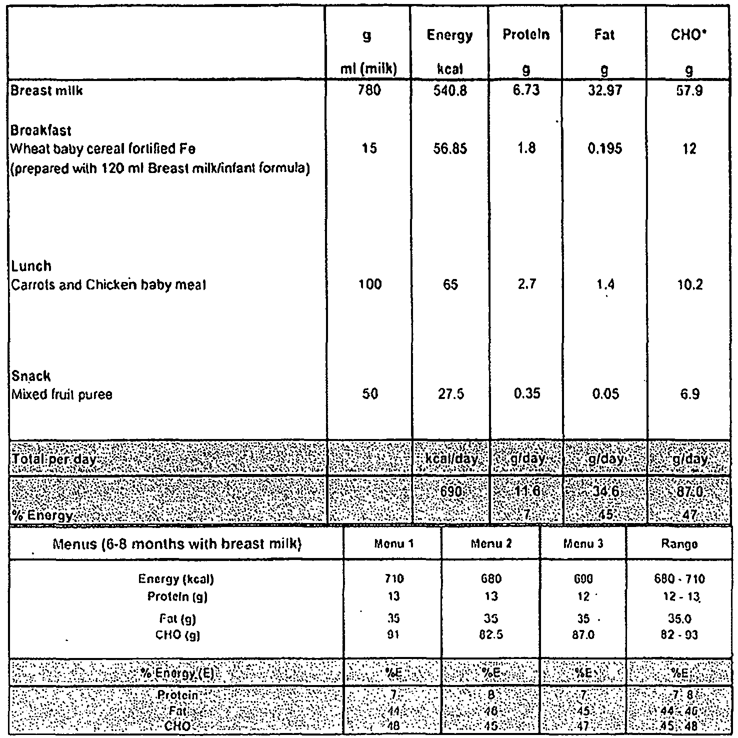

In the following sample diet compositions are provided for the age period of 8-12 months (* = carbohydrates as monosaccharides):

Menu 1:

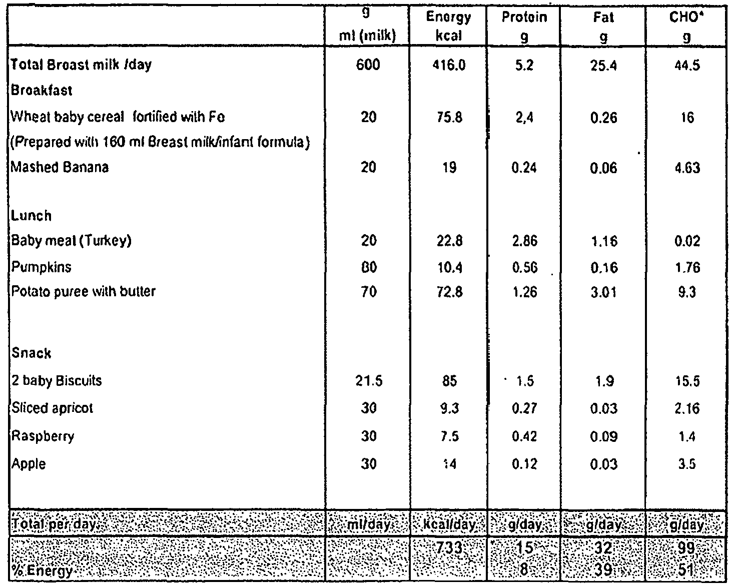

Menu 2:

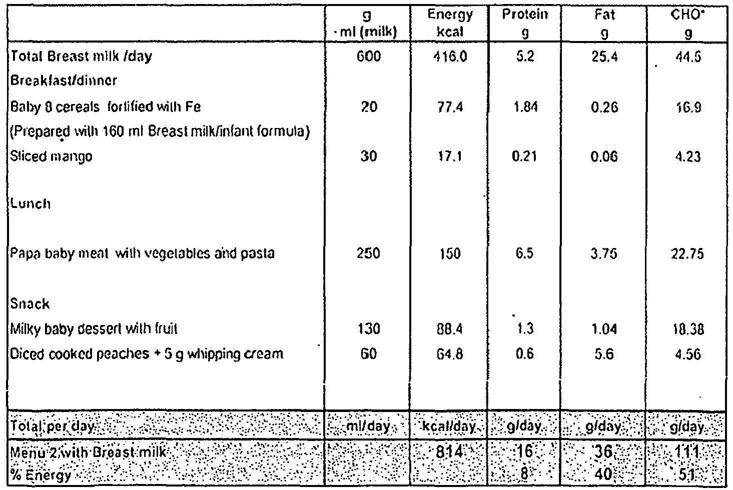

Menu 3:

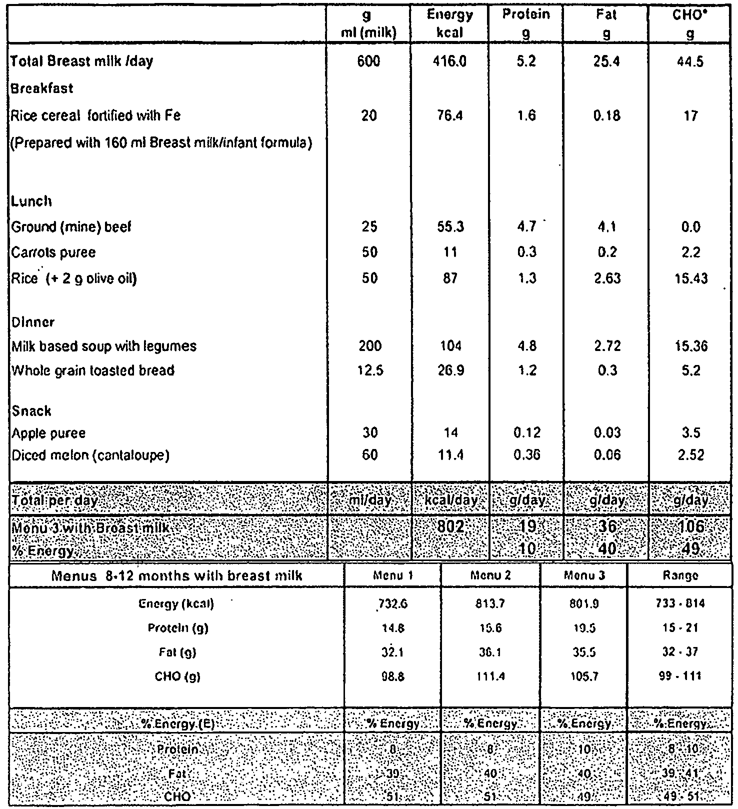

In the following simple diet compositions are provided for the age period of 12-36 months (* = carbohydrates as monosaccharides):

Menu 1:

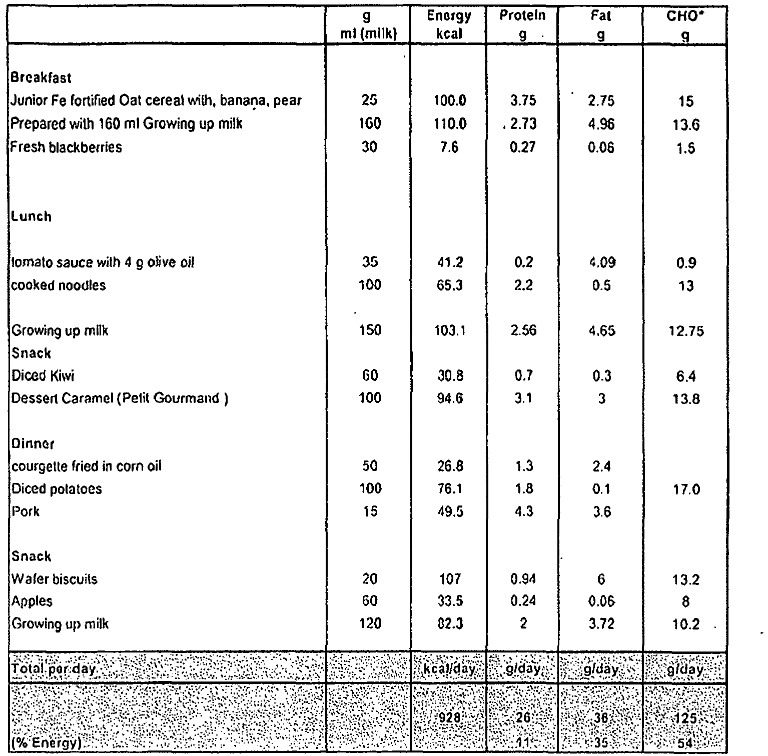

Menu 2:

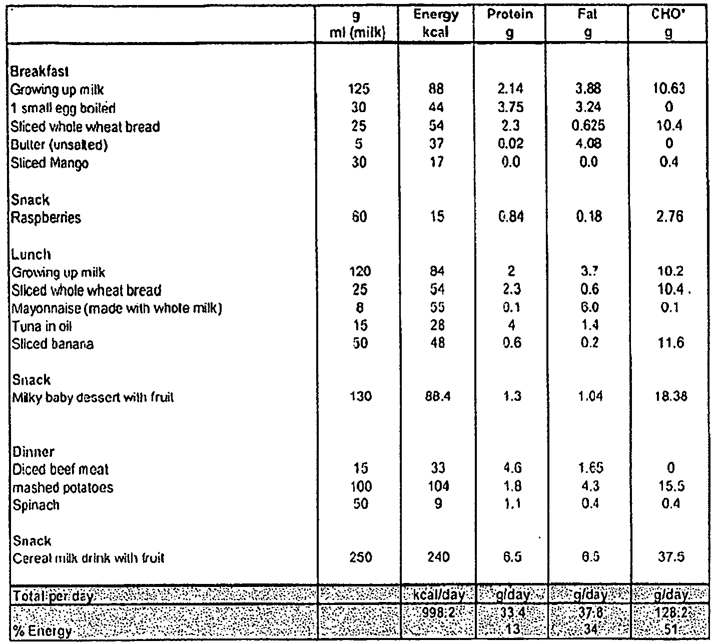

Menu 3:

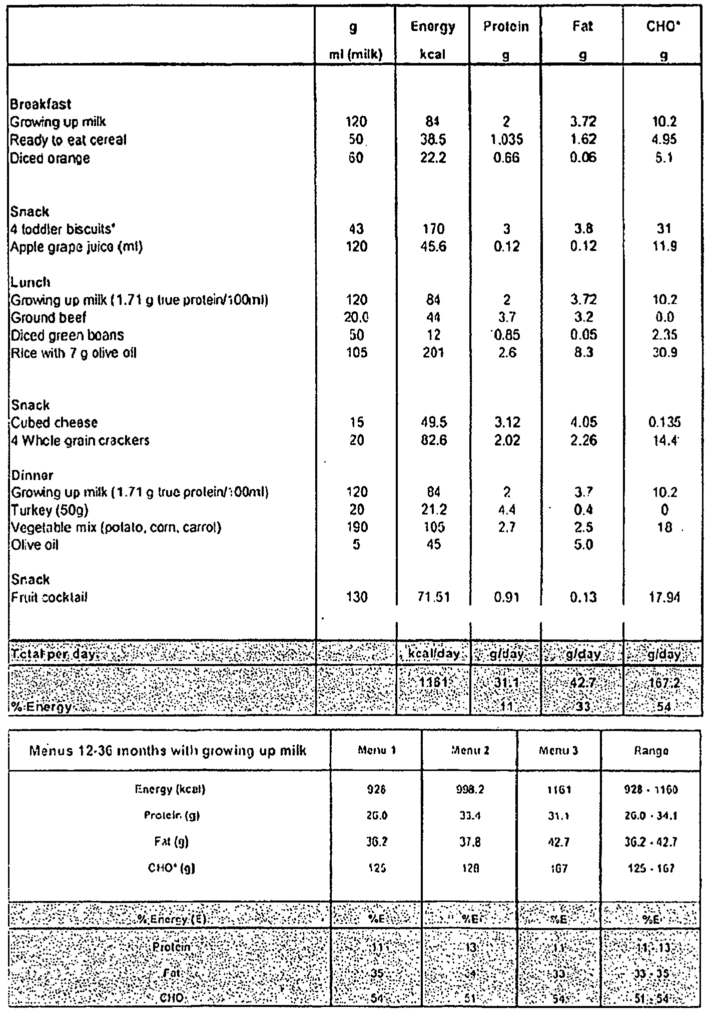

Example 2:

A daily complementary diet for stage 1 comprised:
(i) 100g portion of infant cereal;
(ii) 65g portion of vegetable dish in accordance with the invention; and
(iii) 65g portion of fruit dish in accordance with the invention.

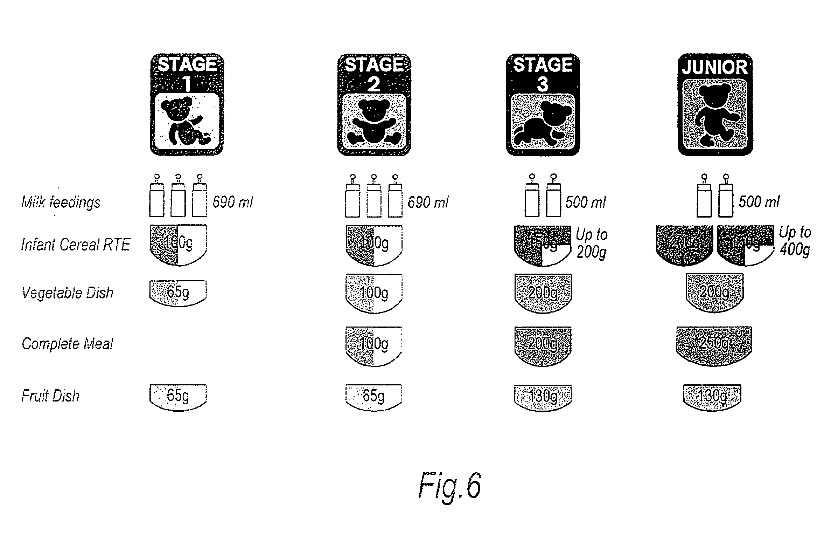

A number of vegetable and fruit dishes were provided in a range of baby food products and were labeled according to their suitability for a stage 1 diet. The feeding plan shown in figure 6 was used to determine which meals could make up the daily food intake for stage 1. In this example, the vegetable dish was a pumpkin based dish and the fruit dish was an apple based dish.

**Table 1: List of ingredients for vegetable dish**

| Ingredient | Amount per 100g (g) |
|---|---|
| POTATO FLAKES | 4.000 |
| Pumpkin frozen | 40.000 |
| Potato 6x6 mm frozen | 10.000 |
| CARROT | 15.000 |
| RAPESEED OIL LOW ERUCIC (CANOLA) | 0.400 |
| SUNFLOWER OIL | 0.400 |
| WATER | 30.200 |

**Table 2: List of ingredients for fruit dish**

| Ingredient | Amount per 100g (g) |
|---|---|
| Apple fresh | 99.95 |
| Vitamin C | 0.05 |

Each dish provided the following nutritional values:

**Table 3: Nutritional values provided by each Stage 1 dish**

| | Kcal/100g | kcal/serving | % daily energy intake |
|---|---|---|---|
| Cereal Dish | 106.0 for RTE pap | 106.00 | 17.0 |
| | 424 for dry powder | | |
| Vegetable Dish | 40.7 | 26.46 | 4.2 |
| Fruit Dish | 53.2 | 34.58 | 5.5 |

The total daily nutritional values provided by the complementary dishes were as follows:

**Table 4: Daily nutritional values provided by the Stage 1 complementary dishes**

| | per day | per 100g | E% (daily energy intake) |
|---|---|---|---|
| Total Protein (g) | 4.3 | 2.8 | 10.4 |
| Total Fat (g) | 3.3 | 2.2 | 18.1 |
| Total CHO (g) | 29.8 | 19.2 | 71.5 |
| Fibres (g) | 3.1 | 2.0 | - |
| Sodium (mg) | 38.1 | 24.6 | - |
| LA (g) | 0.8 | 0.5 | 4.3 |
| ALA (g) | 0.1 | 0.1 | 0.6 |
| LA/ALA ratio | 7.1 | - | - |
| Energy (kcal) | 166.5 | 107.4 | - |

(Wherein CHO represents carbohydrate, LA represents linoleic acid and ALA represents alplta-linolenic acid).

The sum of the daily energy intake provided by the complementary foods was 26.6% (166.5 kcal). The remaining 73.4% (458 kcal) of daily energy intake was provided by milk. This provided a total daily energy intake of 625.04 kcal.

Example 3:

A daily complementary diet for stage 2 comprised:
(i) 100g portion of infant cercal;
(ii) 100g portion of vegetable dish in accordance with the invention;
(iii) 100g portion of a complete meal in accordance with the invention; and
(iv) 65g portion of fruit dish in accordance with the invention.

A number of complete meals, vegetable and fruit dishes were provided in the range of baby food products and were labeled according to their suitability for a stage 2 diet. The feeding plan shown in figure 6 was used to determine which meals could make up the daily food intake for stage 2. In this example, the vegetable dish was garden vegetables and corn based, the complete meal was garden vegetables and lamb based and the fruit dish was apple and raspberry based.

**Table 5: List of ingredients for vegetable dish**

| Ingredient | Amount per 100g (g) |
|---|---|
| CARROT | 30.000 |
| POTATO FLAKES | 3.000 |
| Sweetcom frozen | 10.000 |
| Parsnip frozen | 10.000 |
| RICE SEMOLINA | 1.000 |
| RAPESEED OIL LOW ERUCIC (CANOLA) | 0.400 |
| Fennel frozen | 5.000 |
| SUNFLOWER OIL | 0.200 |
| WATER | 40.400 |

**Table 6: List of ingredients for complete meal**

| Ingredient | Amount per 100g (g) |
|---|---|
| POTATO FLAKES | 4.000 |
| CARROT | 30.000 |
| Lamb frozen | 8.500 |
| RICE SEMOLINA | 2.000 |
| Parsnip frozen | 5.000 |
| Sweetcorn frozen | 3.000 |
| RAPESEED OIL LOW ERUCIC (CANOLA) | 0.600 |
| SUNFLOWER OIL | 0.400 |
| Onion 10 mm frozen | 4.000 |
| WATER | 42.500 |

**Table 7: List of ingredients for fruit dish**

| Ingredient | Amount per 100g (g) |
|---|---|
| Apple fresh | 79.96 |
| Raspberry puree | 19.99 |
| Vitamin C | 0.05 |

Each dish provided the following nutritional values:

**Table 8 : Nutritional values provided by each Stage 2 dish**

| | kcal/100g | kcal/serving | % daily energy intake |
|---|---|---|---|
| Cereal Dish | 106.0 for RTE pap 424 for dry powder | 106.00 | 15.8 |
| Vegetable Dish | 42.6 | 42.6 | 6.4 |
| Complete Meal | 62.6 | 62.6 | 9.3 |
| Fruit Dish | 50.2 | 32.63 | 4.9 |

The total daily nutritional values provided by the complementary dishes were as follows:

**Table 9: Daily nutritional values provided by the Stage 2 complementary dishes**

| | per day | per 100g | E% (daily energy intake) |
|---|---|---|---|
| Total Protein (g) | 7.8 | 2.7 | 12.9 |
| Total Fat (g) | 6.2 | 2.1 | 23.1 |
| Total CHO (g) | 38.5 | 13.3 | 63.9 |
| Fibres (g) | 5.2 | 1.8 | - |
| Sodium (mg) | 75.7 | 26.1 | - |
| LA (g) | 1.2 | 0.4 | 4.6 |
| ALA (g) | 0.2 | 0.1 | 0.6 |
| LA/ALA ratio | 7.3 | - | - |
| Energy (kcal) | 241.1 | 83.1 | - |

The sum of the daily energy intake provided by the complementary foods was 35.9% (241.1 kcal). The remaining 64.1% (428.93 kcal) of daily energy intake was provided by milk. This provided a total daily energy intake of 670.03 kcal.

Example 4:

A daily complementary diet for stage 3 comprised:
(i) 150g portion of infant cereal;
(ii) 200g portion of vegetable dish in accordance with the invention;
(iii) 200g portion of a complete meal in accordance with the invention; and
(iv) 130g portion of fruit dish in accordance with the invention.

A number of complete meal, vegetable and fruit dishes were provided in the range of baby food products and were labeled according to their suitability for a stage 3 diet. The feeding plan shown in figure 6 was used to determine which meals could make up the daily food intake for stage 3. In this example, vegetable dish was garden vegetables and corn based, the complete meal was a pasta, tomato and beef based and the fruit dish was fruit salad based.

**Table 10: List of ingredients for vegetable dish**

| Ingredient | Amount per 100g(g) |
|---|---|
| CARROT | 30.000 |
| POTATO FLAKES | 3.000 |
| Sweetcorn frozen | 10.000 |
| Parsnip frozen | 10.000 |
| RICE SEMOLINA | 1.000 |
| RAPESEED OIL LOW ERUCIC (CANOLA) | 0.400 |
| Fennel frozen | 5.000 |
| SUNFLOWER OIL | 0.200 |
| WATER | 40.400 |

**Table 11: List of ingredients for complete meal**

| Ingredient | Amount per 100g (g) |
|---|---|
| Pasta spaghetti short | 8.000 |
| Beef frozen 14% fat | 8.000 |
| CARROT | 20.000 |
| Tomato puree | 5.000 |
| RAPESEED OIL LOW ERUCIC (CANOLA) | 0.800 |
| SUNFLOWER OIL | 0.400 |
| Bell pepper red 10mm frozen | 4.000 |
| Onion 10 mm frozen | 3.000 |
| Thyme frozen | 0.100 |
| WATER | 50.700 |

**able 12: List of ingredients for fruit dish**

| Ingredient | Amount per 100g (g) |
|---|---|
| Apple fresh | 39.98 |
| Pear William fresh | 14.9925 |
| Peach puree | 14.9925 |
| Banana puree without seeds | 19.990 |
| Apricot puree | 9.995 |
| Vitamin C | 0.05 |

Each dish provided the following nutritional values:

**Table 13: Nutritional values provided by each Stage 3 dish**

| | Kcal/100 g | kcal/serving | % daily energy intake |
|---|---|---|---|
| Cereal Dish | 106.0 | 159.00 | 20.6 |
| Vegetable Dish | 42.6 | 95.2 | 11.1 |
| Complete Meal | 65.9 | 131.8 | 17.1 |
| Fruit Dish | 56.0 | 12.8 | 9.5 |

The total daily nutritional values provided by the complementary dishes were as follows:

**Table 14: Daily nutritional values provided by the Stage 3 complementary dishes**

| | Per day | per 100g | E% |
|---|---|---|---|
| Total Protein (g) | 14.8 | 2.6 | 13.2 |
| Total Fat (g) | 10.9 | 1.9 | 22 |
| Total CHO (g) | 72.4 | 12.3 | 64.8 |
| Fibres (g) | 8.4 | 1.5 | - |
| Sodium (mg) | 142.3 | 25.1 | - |
| LA (g) | 2.3 | 0.4 | 4.6 |
| ALA (g) | 0.4 | 0.1 | 0.7 |
| LA/ALA ratio | 6.5 | - | - |
| Encrgy (kcal) | 447.0 | 78.8 | - |

The sum of the daily energy intake provided by the complementary foods was 58.0% (447.0 kcal). The remaining 42.0% (323.0 kcal) of daily energy intake was provided by milk. This provided a total daily energy intake of 770 kcal.

Example 5:

A daily complementary diet for stage 4 comprised:
(i) 350g portion of infant cereal;
(ii) 200g portion of vegetable dish in accordance with the invention;
(iii) 250g portion of a complete meal in accordance with the invention; and
(iv) 130g portion of fruit dish in accordance with the invention,

A number or complete meals, vegetable and fruit dishes were provided in the range of baby food products and were labeled according to their suitability for a stage 4 diet. The feeding plan shown in figure 6 was used to determine which meals could make up the daily food intake for stage 4. In this example, the vegetable dish was a garden vegetables and corn dish, the complete meal was a pasta, tomato and beef dish and the fruit dish was a fruit salad dish.

**Table 15: List of ingredients for vegetable dish**

| Ingredient | Amount per 100g (g) |
|---|---|
| CARROT | 30.000 |
| POTATO FLAKES | 3.000 |
| Sweetcorn frozen | 10.000 |
| Parsnip frozen | 10.000 |
| RICE SEMOLINA | 1.000 |
| RAPESEED OIL LOW ERUCIC (CANOLA) | 0.400 |
| Fennel frozen | 5.000 |
| SUNFLOWER OIL | 0.200 |
| WATER | 40.400 |

**Table 16: List of ingredients for complete meal**

| Ingredient | Amount per 100g (g) |
|---|---|
| Pasta spaghetti short | 8.000 |
| Beef frozen 14% fat | 8.000 |
| CARROT | 20.000 |
| Tomato puree | 5.000 |
| RAPESEED OIL LOW ERUCIC (CANOLA) | 0.800 |
| SUNFLOWER OIL | 0.400 |
| Bell pepper red 10mm frozen | 4.000 |
| Onion 10 mm frozen | 3.000 |
| Thyme frozen | 0.100 |
| WATER | 50.700 |

**Table 17: List of ingredients for fruit dish**

| Ingredient | Amount per 100g (g) |
|---|---|
| Apple fresh | 39.98 |
| Pear William fresh | 14.9925 |
| Peach purce | 14.9925 |
| Banana puree without seeds | 19.990 |
| Apricot purce | 9.995 |
| Vitamin C | 0.05 |

Each dish provided the following nutritional values:

**Table 18: Nutritional values provided by each Stage 4 dish**

| | kcal/100g | kcal/serving | % daily energy intake |
|---|---|---|---|
| Cereal Dish | 106.0 | 371 | 35.7 |
| Vegetable Dish | 42.6 | 85.2 | 8.2 |
| Complete Meal | 65.9 | 164.75 | 15.8 |
| Fruit Dish | 56.0 | 72.8 | 7.0 |

The daily nutritional values provided by the complementary dishes were as follows:

**Table 19: Daily nutritional values provided by the Stage 4 complementary dishes**

| | per day | per 100g | E% |
|---|---|---|---|
| Total Protein (g) | 23.6 | 3.5 | 13.6 |
| Total Fat (g) | 17.2 | 2.6 | 22.4 |
| Total CHO (g) | 110.6 | 16.6 | 64.0 |
| Fibres (g) | 9.8 | 1.5 | - |
| Sodium (mg) | 215.0 | 32.2 | - |
| LA (g) | 3.4 | 0.5 | 4.5 |
| ALA (g) | 0.5 | 0.1 | 0.7 |
| LA/ALA ratio | 6.8 | - | - |
| Energy (kcal) | 691.7 | 103.6 | - |

The sum of the daily energy intake provided by the complementary foods was 66.5% (691.7 kcal). The remaining 33.5% (348.3 kcal) of daily energy intake was provided by milk. This provided a total daily energy intake of 1040 kcal.

References

Barker DJ, Clark PM. Fetal undernutrition and disease in later life. Rev Reprod 1997;2:105-112.

Law CM, Barker DJ, Osmond C, Fall CH, Simmonds SJ. Early growth and abdominal fatness in adult life. J Epidemiol Community Health, 1992;46 184-18.

Barker DJ. Outcome of low birthweight. Horm Res 1994;42:223-230.

Hoet JJ, Hanson MA. Intrauterine nutrition: its importance during critical periods for cardiovascular and endocrine development. J Physiol (Lond) 1999;514:617-627.

Ozanne SE, Hales CN. The long-term consequences of intra-uterine protein malnutrition for glucose metabolism. Proc Nutr Soc 1999;58:615-619.

Langley-Evans SC, Sherman RC, Welham SJ, Nwawu MO, Gardner DS, Jackson AA. Intrauterine programming of hypertension: the role of the renin-angiotensin system. Biochem Soc Trans 1999;27:88-93.

Tycko B, Ashkenas J. Epigenetics and its role in disease. J. Clin Invest, 2000; 105:245,246.

Patel M.S, Vadlamudi S.P and Johanning G.L, Overview of pup in a cup model: hepatic lipogenesis in rats artificially reared on a high-carbohydrate formula J.Nutr, 1993,123:373-377.

Hiremagalur B.K, Vadlamudi S, Johanning G.L and Patel, M.S, Long-term effects of feeding high carbohydrate diet in pre-weaning period by gastrostomy: a new rat model for obesity. Inter J Obesity, 1993,17:495-502.

Song F, Srinivasan M, Aalinkeel R, Patel MS. Use of cDNA Array for identification of genes induced in islets of suckling b rats by a high-carbohydrate nutritional intervention. Diabetes 2001;50:2053-2060.

Aalinkeel R, Srinivasan M. Song F and Patel MS. Programming into adulthood of islet adaptations induced by early nutrition in the rat. Am J Physiol Endocrinol Metab, 2001, 281:E640-648.

Michaelsen FK and Jorgensen M. Dietary fat content and energy density during infancy and childhood, Eur J Clin Nutr, 1995, 49: 467-483.

Lapinleimu.H, Viikari J, Jokinen E, Salo P, Routi T, Leino A, Ronnemaa T, Seppanen R, Valimaki I, Simell O, Prospective randomised trial in 1062 infants of diet low in saturated fat and cholesterol. Lancet. 1995;345:471-476

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

## Claims

1. A range of shelf-stable baby food products comprising two or more food products suitable for each stage of infant development, the products having an optimal energy profile for meeting the nutritional needs of an infant at a specific stage of infant development, wherein two or more baby food products from the range may be combined to provide an optimal energy profile for meeting the nutritional needs of an infant at a particular stage of infant development.

2. The range of claim 1, wherein (a) the stages of infant development are selected from:
(i) from about 4 to about 6 months of age (Stage 1);
(ii) from about 6 to about 8 months of age (Stage 2);
(iii) from about 8 to about 12 months of age (Stage 3); and
(iv) from about 12 to about 36 months of age (Stage Junior); optionally
wherein the total optimum daily energy intake for stage 1 is between about 530 and about 700 kcal/day, preferably about 625 kcal/day, for stage 2 is between about 620. and about 720 kcal/day, preferably about 670 kcal/day, for stage 3 is between about 680 and about 930 kcal/day, preferably about 770 kcal/day, and for stage junior is between about 810 and about 1180 kcal/day, preferably about 1040 kcal/day; or (b) two or more baby food products from the range may be combined with the daily milk intake of an infant to provide an optimal daily energy intake of an infant at a particular stage of infant development; or (c) two or more baby food products from the range provide a daily energy intake for stage 1 of between about 68 and about 238 kcal/day, preferably about 167 kcal/day, for stage 2 of between about 158 and about 258 kcal/day, preferably about 243 kcal/day, for stage 3 of between about 345 and about 595 kcal/day, preferably about 448 kcal/day, and for stage junior of between about 475 and about 845 kcal/day, preferably about 693 kcal/day; optionally wherein the difference between the daily energy intake provided by the two or more baby food products and the optimum daily energy intake for each stage of infant development is provided by the daily milk intake of an infant; or (d) a daily intake of baby food products from the range has an average energy density of at least about 0.6 kcal/g, preferably at least about 0.67 kcal/g, most preferably about 1 kcal/g; or (e) one or more daily feeding schedules are provided which comprise suggested combinations of baby food products from the range of baby food products, said combinations providing an optimal energy profile for meeting the nutritional needs of an infant at a particular stage of infant development; optionally one or more daily feeding schedules are provided for each stage of infant development; or (f) the total optimum daily fat intake for stage 1 is about 45%E, for stage 2 is between about 43%E and about 45%E, for stage 3 is between about 40%E and about 43%E, and for stage junior is between about 30%E and about 40%E; or (g) two or more baby food products from the range provide a daily fat intake for stage 1 of between about 18%E and about 27%E, for stage 2 of between about 22%E and about 29%E, for stage 3 of between about 21%E and about 36%E, and for stage junior of between about 22%E and about 40%E; optionally wherein the difference between the daily fat intake provided by the two or more baby food products and the optimum daily fat intake for each stage of infant development is provided by the daily milk intake of an infant.

3. The range of claim 1 or 12, wherein the baby food products have a protein density of between about 1.5g/100kcal and about 3.2g/100kcal.

4. The range of any of claims 1 to 3, wherein (a) the baby food products comprise complete meals or incomplete meals; optionally wherein a complete meal comprises two or more incomplete meals; or (b) each baby food product comprises a serving size of between about 65g and about 350g; or (c) each baby food product comprises a serving size of between about 65g and about 100g for stage 1, of between about 65g and about 100g for stage 2, of between about 130g and about 200g for stage 3, and between about 130g and about 350g for stage junior.

5. A feeding regime for meeting the nutritional needs of an infant during different stages of infant development, the regime comprising administering to an infant a range of baby food products of any of claims 1 to 4.

6. The use of a range of any of claims 1 to 4, in the manufacture of a medicament for the prevention or treatment of a nutritionally related disorder in an infant; optionally wherein the nutritionally related disease is selected from obesity, malnutrition, diabetes and heart disease.

7. A kit of at least 3 individual nutritional compositions wherein each nutritional composition represents an individual meal for children age 6-36 month, comprising a macronutrient content that gradually changes in a substantially straight line from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months.

8. The kit in accordance with claim 7 wherein (i) the diet compositions are daily compositions; or (ii) the total energy content of the composition is gradually changing in the form of a substantially straight line from a composition that comprises about 670-715 kcal/day for children at the age of 6 months to a composition that comprises about 1000-1200 kcal/day for children at the age of 36 months; or (iii) the content of the diet composition is adjusted (a) daily, based on the age of the child and the corresponding optimal fat content and carbohydrate content of the composition, (b) weekly, based on the age of the child and the corresponding optimal fat content and carbohydrate content of the composition, or (c) monthly, based on the age of the child and the corresponding optimal fat content and carbohydrate content of the composition; or (iv) the content of the diet composition is adjusted stepwise, based on the age of the child and the corresponding optimal fat content and carbohydrate content of the composition; optionally wherein (a) the composition is adjusted in 3-10 steps during the age of 6-36 months; or (b) the composition is adjusted in 3-4 steps during the age of 6-36 months; or (v) the content of the diet composition is adjusted stepwise, based on the age of the child and the corresponding optimal fat content and carbohydrate content of the composition, at the age of the child of 6, 8, 12, 18, 24 and 36 months; or (vi) the content of the diet composition is adjusted also with respect to the total calorie content based on the age of the child and the corresponding optimal calorie content of the composition.

9. The kit in accordance with claim 7, comprising
at least one diet composition that comprises about 44-46% energy from fat and about 47-49% energy from carbohydrates for children age 6-8 months;
at least one diet composition that comprises about 39-41% energy from fat and about 49-52% energy from carbohydrate for children age 8-12 months; and
at least one diet composition that comprises about 34-35% energy from fat and about 51-53% energy from carbohydrate for children age 12-36 months; optionally wherein the at least one diet composition comprises about 670-715 kcal/day during the age of 6-8 months, about 715-850 kcal/day for children age 8-12 months, and about 750-1200 kcal/day for children age 12-36 months.

10. The kit in accordance with claim 7, wherein the diet compositions further comprise a protein source.

11. The kit in accordance with claim 7, wherein the content of carbohydrates in the compositions is gradually changing from a composition that comprises about 48% energy from carbohydrates for children at the age of 6 months to a composition that comprises about 53% energy from carbohydrates for children at the age of 36 months.

12. The kit in accordance with claim 7, comprising diet compositions for at least (a) one day, (b) three days, (c) a week, or (d) a month.

13. The kit in accordance with claim 7, wherein the diet compositions represent a total diet.

14. A method for reducing the risk of obesity in children 6-36 months old comprising the steps of administering a series of diet compositions, wherein the macronutrient content of the compositions is gradually changing from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months.

15. A method for developing a mealplan for children age 6-36 months comprising a macronutrient content that gradually changes in a substantially straight line from a composition that comprises about 40-50% energy from fat and about 40-49% energy from carbohydrates for children at the age of 6 months to a composition that comprises about 30-35% energy from fat and about 50-55% energy from carbohydrates for children at the age of 36 months.
